# EUROPEAN PATENT APPLICATION

(11) **EP 2 543 326 A1**
(43) Date of publication of application: **09.01.2013**
(21) Application number: 11075150.0
(22) Date of filing: 05.07.2011
(51) Int. Cl.: A61B 17/16, A61L 31/02, C22C 38/00

(54) **Bone removal tools**

(71) Applicant: Arunjunai, Latha, 7326 SE Apeldoorn (NL)
(72) Inventor: Arunjunai, Latha, 7326 SE Apeldoorn (NL)

(57) **Abstract**

The present invention relates to a tool that is used for the removal of material from bones as required in orthopaedic surgery and other forms of surgery. The present invention especially relates to a corrosion resistant and ductile tool for the removal of bone material from human bones as required in orthopaedic surgery and other forms of surgery. A tool according to the present invention can reduce the occurrence of adverse events during surgical procedures.

## Description

FIELD OF THE INVENTION

The present invention relates to a tool that is used for the removal of material from bones as required in orthopaedic surgery and other forms of surgery. The present invention especially relates to a corrosion resistant and ductile tool for the removal of bone material from human bones as required in orthopaedic surgery and other forms of surgery.

BACKGROUND OF THE INVENTION

There are a relatively high number of adverse events which occur during surgery due to breakage of drill bits during bone drilling. One source that gives some insight in the frequency and the nature of such events is the MAUDE (Manufacturer and User Facility Device Experience) database of the US Food and Drugs Administration. In most cases the breakage leads at least to a delay during surgery. In some cases when for example the tip of a drill bit that is broken off cannot be retrieved from inside the bone the breakage can put a proper execution of the intended procedure at risk.

So far drill bits for bone drilling are manufactured from hardened martensitic grades of stainless steel. This material is characterized by an inherently low ductility, which explains the drill bit breakage problems as reported. The present invention was triggered by these problems and provides a solution which can prevent such adverse events.

SUMMARY OF THE INVENTION

For the sake of convenience the drill bits according to the invention will often be referred to as drill bits or orthopaedic drill bits in this descriptive section.

In spite of all the adverse events that occur with the conventional orthopaedic drill bits made of martensitic stainless steel, the manufacturers of these components have not been able yet to provide an acceptable alternative. The present invention relates to an attractive alternative for the conventional orthopaedic drill bits. The present invention relates to orthopaedic drill bits made from austenitic stainless steel instead of martensitic stainless steel.

Bone drilling should be done preferably with a drill with sufficient cutting action (sharp and hard cutting edges) using a high axial drill speed in order to limit the localised bone temperature increase which occurs during drilling. An increase of the bone temperature above the critical value of 50°C can result in thermal necrosis, irreversible death, of bone cells.

A high axial drill speed requires the exertion of a higher axial drilling force which in case of an unintended lateral movement may increase the bending and/or buckling stress in a drill bit. Such bending and/or buckling stresses may then result in fracturing of the drill bit if the drill bit lacks sufficient ductility. Fracturing of conventional martensitic drill bits may also occur when the drill bit comes into contact with implants and other metal or ceramic objects during drilling.

When cannulated drill bits according to prior art are used the risk of fracturing is even higher than for solid drill bits.

From the above it will be clear that the ideal orthopaedic drill bit shall have a combination of a high hardness, a high strength and a high ductility. In addition, such a device also requires corrosion resistance. Even for disposable drill bits sufficient corrosion resistance is required in order to prevent the formation of corrosion products during storage, atmospheric exposure and exposure to physiological conditions in the human body during the performance of a surgical procedure. For reusable orthopaedic drill bits a higher corrosion resistance, against both general and localised forms of corrosion, is required due to the exposure to cleaning and sterilisation treatments.

In view of the above-mentioned combination of properties required for an ideal orthopaedic drill bit, the use of austenitic stainless steel (which has a low hardness compared to martensitic grades of stainless steel) was never considered to be a realistic option for orthopaedic drill bits. Only if the corrosion resistance and high ductility of austenitic stainless steels could be combined with a high hardness this would result in an acceptable material for orthopaedic drill bits.

From the literature and the inventor's own experience it is known that numerous attempts have been made over the past decades to treat austenitic stainless steels in order to increase the hardness. Treatments such as nitriding, carburising, conventional ion-nitriding, carbonitriding, etc. have been used for that purpose. These treatments involve the diffusion of nitrogen (nitriding), carbon (carburisation) or both carbon and nitrogen (carbonitriding) into the surface layers of the steel. While such conventional treatments result in a hardened diffusion layer at the surface of austenitic stainless steel, they invariably resulted in grain boundary sensitisation in the austenitic stainless steel. The nitrogen and/or carbon react with the chromium that is present in the austenitic stainless steels to form chromium nitrides and/or chromium carbides. Since the grain boundaries form a preferred diffusion path, the formation of chromium nitrides and/or chromium carbides takes place preferentially in a zone along the grain boundaries. This zone is then depleted of chromium. The chromium content of the steel in this zone drops below the chromium level required for maintaining corrosion resistance. Therefore, austenitic stainless steel that is subjected to the above diffusion treatments will lose its corrosion resistance which will become apparent in various corrosive environments.

In specific corrosive media the corrosive attack along the grain boundaries, commonly referred to as intergranular corrosion, can be quite severe, even to the extent that the affected material becomes unsuitable for further use.

When austenitic stainless steels are subjected to conventional case hardening treatments that deliberately increase the concentration of elements such carbon and/or nitrogen in an outer layer of the stainless steel to a level in the order of weight percents, the use of so-called stabilised or low-carbon (L-grade) austenitic stainless steel does not provide any solution in preventing grain boundary sensitisation.

In addition to trials with these diffusion treatments, it has been tried to apply hard coatings e.g. chrome plating, PVD (physical vapour deposition) and CVD (chemical vapour deposition) coatings to austenitic stainless steel drill bits in order to obtain sufficient wear resistance for bone drilling. For several cutting tools titanium nitride (TiN) is one of the favourite coatings. Therefore, TiN is sometimes also applied to surgical tools by certain manufacturers.

The problem with hard coatings on austenitic stainless steel is the bonding between the hard coating and the austenitic stainless steel substrate material which is inherently soft. The great difference between the coating and the substrate in terms of the mechanical properties, particularly the hardness and ductility, gives rise to disbonding. The coating, due to its very limited thickness has no load bearing capabilities and depends on the load bearing properties of the substrate. In case of austenitic stainless steel the latter is relatively low. Hence, austenitic stainless steel will readily deform when a high load is exerted on the surface. This degree of deformation cannot be followed by the hard coating which consequently cracks and usually flakes off. It is obvious that flakes of hard coatings can have harmful effects when released inside the human body.

Therefore, hard coatings don't provide the required safe solution to allow orthopaedic drill bits to be made from austenitic stainless steels.

US patent 5,269,798 describes a surgical cutting instrument with movable inner and outer tubular members made from austenitic stainless steel whereby the outer surface of the inner member is case hardened to prevent galling between the outer and inner case. Galling or adhesive wear is a common problem of austenitic stainless steel.

The Hardcor case hardening process described in this patent is a commercially available process (be it under a different name nowadays) which produces a hardened case depth of 22 to 33 micrometers with a surface hardness in the range of 1000 to 1200 Hv 0,05. The commercial treatment described in that patent results in a hardened case without the grain boundary sensitisation effect that is present after conventional case hardening treatments such as nitriding, carburising or carbonitriding. The Hardcor case hardening process is a carburising process that according to published information is performed at a temperature below the temperature range in which chromium reacts with the carbon that diffuses into the material. The hardening effect of this process is a result of high compressive stresses that are formed by carbon atoms which are interstitially dissolved in the crystal lattice of the austenitic stainless steel. The fact that according to US patent 5,269,798 this treatment prevents galling between the inner and outer member of a surgical cutting instrument does not make the use of this or a comparable treatment for orthopaedic drill bits an obvious step. The contrary is true. It probably seems obvious to orthopaedic drill bit designers that a hardened case of 20 to 40 micrometers cannot provide sufficient load bearing strength to compensate the low hardness of the austenitic substrate material which probably explains why the well-established manufacturers of orthopaedic drill bits never introduced austenitic stainless steel drill bits.

So, the curiosity, inventiveness and persistence of the originator of the present invention, relating to austenitic stainless steel orthopaedic drill bits, to apply a novel case hardening treatment such as the previously mentioned Hardcor treatment to drill bits for bone drilling made from austenitic stainless steel constitutes an inventive step as he pursued a route that most others either did not consider at all or considered too improbable to even try.

The present invention comprises orthopaedic drill bits made from austenitic stainless steel and therefore solving the problem of frequent adverse events due to fracturing of the conventional martensitic orthopaedic drill bits as is still the case in the year of the writing of this patent application as can be concluded from the information in the FDA MAUDE database.

The invention includes the route of treating the austenitic stainless steel drill bits by means of a case hardening process that does not, or only to a negligible extent, result in grain boundary sensitisation of the austenitic stainless steel. In addition to the Hardcor low temperature case hardening treatment, other treatments, such as improved modified ion-nitriding processes, that mimic the attractive effects of the Hardcor treatment are now becoming available.

A wide range of austenitic stainless steels is suitable for the manufacturing of drill bits according to the present invention. In addition to the common austenitic stainless steels, belonging to the group that is usually referred to as the AISI 300 stainless steels, iron based austenitic alloys such as AISI 660 (also known as A286) are also included in the range of austenitic stainless steels covered by the present invention. When a higher base hardness is desired austenitic precipitation hardening stainless steels such as 17-7 PH can be considered. It should be taken into account that the corrosion resistance of the latter material is inferior to that of most of the common austenitic stainless steels from the AISI 300 series.

Of the AISI 300 series the grades which contain molybdenum such as 316 and 316L are the preferred ones when considering reusable orthopaedic drill bits according to this invention, since these exhibits a higher corrosion resistance than the grades without molybdenum such as 304.

In view of the enhancement of the strength and hardness by cold deformation of austenitic stainless steel an embodiment of the drill bit as per the present invention involves the use of cold drawn austenitic stainless steel wire or bar. However, when the best corrosion resistance is to be obtained the amount of cold work shall be limited particularly when austenitic stainless steels with a relatively low nickel content are used. The lower the nickel content (within the specification range) of the specific austenitic stainless steel the higher the hardness increase due to cold deformation may be. However, in such a case the extra hardening effect is due to the fact that the relatively low nickel content results in a lower austenite stability which in turn leads to transformation of some austenite into deformation martensite (ε-martensite).

The presence of deformation martensite has a negative effect on the corrosion resistance of the treated drill bit.

Apart from the stainless steels classified as austenitic stainless steels (the AISI 300 series) there are also stainless steels which are approximately 50% austenitic and 50% ferritic. These stainless steels are commonly known as duplex stainless steel. The most common grades of duplex stainless steels are generally referred to as 2205 and 2507. The 2205 has a chromium content of approximately 22% and a nickel content of 5%, while the 2507 grade has a chromium content of 25% and a nickel content of 7%. Mainly due to their lower nickel content the duplex stainless steels have a lower price than the austenitic stainless steels and are easier to machine.

The high ductility and work hardening of austenitic stainless steels makes such steels difficult to machine. During machining the chips don't break off easily, leading to undesirable effects. For this reason in some grades of austenitic stainless, such as AISI 303, sulphur is added to the steel wherein the resulting sulphides have an embrittling effect on the chips that are formed during machining, hence resulting in easier breakage 30 of the chips. These grades are also referred to as free-machining grades.

Since duplex stainless steels are roughly half ferritic the machining characteristics are much more favourable than those of austenitic stainless steels. Therefore, the machining cost for duplex stainless steel is lower than the cost of machining austenitic stainless steel.

In most of the common corrosive environments duplex stainless steels exhibit a corrosion resistance which is not significantly inferior to that of austenitic stainless steels. The resistance against chloride stress corrosion cracking of duplex stainless steels is even better than that of austenitic stainless steels thanks the partially ferritic microstructure.

The strength, base hardness and ductility of duplex stainless steel makes it an attractive candidate material for the manufacturing of bone drills, provided the shortcomings of in particular the austenitic constituent can be eliminated. Trials with conventional case hardening treatments of duplex stainless steels resulted in the same problems that are experienced upon treatment of austenitic stainless steels, i.e. the formation of compounds of chromium and nitrogen and/or carbon, with the pertaining detrimental effect on the properties of the material.

However, the introduction of treatments such as the Hardcor treatment as described earlier have opened the possibility for surface hardening of duplex stainless steels without undesirable side effects. During the development of drill bits according to the present invention tests were carried out with low temperature case hardening of orthopaedic drill bits made from duplex stainless steel resulting in satisfactory bone drilling characteristics. Therefore, the scope of the present invention that at first was limited to the use of austenitic stainless steels could be expanded to include drill bits for the drilling of bones as required in orthopaedic and other forms of surgery, made from substantially austenitic stainless steels.

The present invention covers all the common types of drill bits that are used in bone drilling, such as classic solid drill bits, step drill bits for bone drilling like for example two-step or two phase drill bits and cannulated drill bits.

During the process of providing a proof of concept for the drill bits according to the present invention, the basic concept of the present invention, i.e. the manufacturing of tools for the removal of bone material from human bones from a substantially austenitic stainless steel, was also applied successfully to several other surgical bone machining tools, such as acetabular reamers, bone-cutting saw blades, taps and bone rasps. Although, contrary to drill bits, the conventional martensitic versions of these tools in general don't cause breakage problems, they often do exhibit corrosion problems as a result of cleaning and sterilisation. As demonstrated by the present invention the manufacturing of these tools from an austenitic stainless steel or a duplex stainless steel with a subsequent low temperature case hardening treatment provides the required mechanical properties for the removal of bone material while preserving the corrosion resistance of the austenitic or duplex stainless steel.

All examples given in this descriptive section are intended to be nonlimiting, and are provided in order to help convey the scope of the invention.

## Claims

1. A tool that is used for the removal of material from bones as required in orthopaedic surgery and other forms of surgery, **characterized in that** said tool is made out of stainless steel with a minimum of 40% austenite with a surface hardness of minimum 850 Hv 0.05 obtained due the presence of compressive stresses developed by the interstitial dissolution of carbon atoms in the austenite.

2. A tool according to claim 1, **characterized in that** said tool is made out of austenitic stainless steel.

3. A tool according to claim 1, **characterized in that** said tool is made out of duplex stainless steel.

4. A tool according to one or more of the above mentioned claims, **characterized in that** the carbon atoms in the said tool are dissolved in the steel at a temperature level below the temperature at which chromium carbide can form by a reaction between chromium in the stainless steel and the carbon that is brought into the stainless steel.

5. A tool according to one or more of the above mentioned claims, **characterized in that** said tool is a drill bit for drilling holes in bones as required for orthopaedic surgery and other forms of surgery

6. A tool according to one or more of the above mentioned claims, **characterized in that** said tool is a reamer to remove material from holes in bones as required for orthopaedic surgery and other forms of surgery

7. A tool according to one or more of the above mentioned claims, **characterized in that** said tool is a rasp to remove material from bones as required for orthopaedic surgery and other forms of surgery

8. A tool according to one or more of the above mentioned claims, **characterized in that** said tool is a tap to make screw threads in holes in bones as required for orthopaedic surgery and other forms of surgery

9. A tool according to one or more of the above mentioned claims, **characterized in that** said tool is a blade to cut bones as required for orthopaedic surgery and other forms of surgery

10. A tool according to one or more of the above mentioned claims, **characterized in that** said tool is made out of stainless steel with a minimum of 40% austenite and that the steel has been work hardened, whereby the strength and the bulk hardness of the material has been increased compared to the non work hardened condition.
